# EUROPÄISCHE PATENTSCHRIFT

(11) **EP 2 194 378 B1**
(45) Veröffentlichungstag und Bekanntmachung des Hinweises auf die Patenterteilung: **27.02.2013**
(21) Anmeldenummer: 08020879.6
(22) Anmeldetag: 02.12.2008
(51) Int. Cl.: A61B 5/145

(54) **Handgerät zur Messung einer Analytkonzentration einer Körperflüssigkeitsprobe**
Hand tool for measuring the analyte concentration in a body fluid sample
Appareil manuel destiné à la mesure d'une concentration en analytes dans un échantillon de liquide corporel

(43) Veröffentlichungstag der Anmeldung: 09.06.2010
(73) Patentinhaber: Roche Diagnostics GmbH, 68305 Mannheim (DE); F.Hoffmann-La Roche AG, 4070 Basel (CH)
(72) Erfinder: Porsch, Ulrich, 69469 Weinheim (DE); Steiger, Bernd, 67354 Römerberg (DE); Albrecht, Gertrud, 68239 Mannheim (DE); Wittmann, Uwe, 68623 Lampertheim (DE)
(74) Vertreter: Twelmeier Mommer & Partner

(56) Entgegenhaltungen:
- EP-A- 1 987 766
- WO-A-2007/030457
- US-A1- 2005 103 351
- STEVENS M W: "Unification of relative time frames for digital forensics" 20040901, Bd. 1, Nr. 3, 1. September 2004 (2004-09-01), Seiten 225-239, XP004552533

## Beschreibung

Die Erfindung betrifft ein Handgerät zur Messung einer Analytkonzentration einer Körperflüssigkeitsprobe mit den im Oberbegriff des Anspruchs 1 angegebenen Merkmalen. Ein derartiges Handgerät ist aus der WO 2007/030457 A1 bekannt und dient zur quantitativen Analyse von Körperflüssigkeiten, beispielsweise Urin, Blut und interstitieller Flüssigkeit, nämlich zur Messung der Konzentration medizinisch bedeutsamer Analyten wie beispielsweise Lactat, Cholesterol und insbesondere Glucose.

Solche Handgeräte können von Benutzern ständig mitgeführt werden und werden insbesondere von Diabetikern benötigt, die mehrmals täglich an einer Probe aus Blut und/oder interstitieller Flüssigkeit eine Messung der Glukosekonzentration vornehmen müssen.

Die erzeugten Messwerte können in einem Messwertspeicher des Handgeräts abgespeichert werden, um später auf ein externes Auswertegerät, beispielsweise den PC eines Arztes, übertragen zu werden. Durch eine Auswertung von über einem längeren Zeitraum gewonnenen Messwerten kann die medizinische Behandlung von chronischen Krankheiten wie Diabetes optimiert werden. Moderne Handgeräte enthalten deshalb eine Uhr, damit bei einer späteren Auswertung von Messergebnissen unter Berücksichtigung einer gespeicherten Zeitinformation der zeitliche Verlauf der Analytkonzentration untersucht werden kann.

Die zeitliche Relation zwischen den einzelnen Messwerten kann bei einem Verstellen der Uhr eines Handgeräts verfälscht werden. Dies lässt sich vermeiden, indem Handgeräte mit einer internen Uhr versehen werden, die sich von einem Benutzer nicht verstellen lässt, wie es aus der DE 197 33 445 A1 bekannt ist. Da medizinisch relevante Analytkonzentrationen üblicherweise vom Tagesrhythmus bedingten Schwankungen unterliegen, ist es jedoch wünschenswert, die Uhr eines Handgeräts gegebenenfalls verstellen zu können, beispielsweise um eine Umstellung zwischen Sommerzeit und Winterzeit oder bei Reisen in andere Zeitzonen eine Anpassung an die lokale Tageszeit vornehmen zu können. Um eine Auswertung von über einen längeren Zeitraum gewonnenen Messwerten nicht zu verfälschen, müssen derartige Zeitumstellungen erfasst werden.

Aus der WO 2007/030457 ist ein Handgerät bekannt, bei dem Messwerte sowie Datum und Uhrzeit der dem Messwert zugrunde liegenden Messungen zu Messwertdatensätzen zusammengefasst und fortlaufend in einem Messwertspeicher gespeichert werden. Bei einem Verstellen der Uhr werden Betrag und Richtung der Verstellung in einem dafür vorgesehenen Speicher (buffer log) gespeichert, so dass die betreffende Information bei einer späteren Auswertung der Messwerte berücksichtigt werden kann.

Aufgabe der vorliegenden Erfindung ist es, einen Weg aufzuzeigen, wie mit geringerem Aufwand die Auswertung einer Serie von Messwerten eines Handgeräts ohne Verfälschung durch eventuell erfolgte Zeitumstellungen ermöglicht werden kann.

Diese Aufgabe wird durch ein Handgerät mit den im Anspruch 1 angegebenen Merkmalen gelöst. Vorteilhafte Weiterbildungen der Erfindung sind Gegenstand der Unteransprüche.

Ein erfindungsgemäßes Handgerät erzeugt bei jedem Verstellen der Uhr einen Zeitkorrekturdatensatz, der Betrag und Richtung der Verstellung angibt und zusammen mit Messwertdatensätzen, die jeweils einen Messwert, sowie Datum und Uhrzeit ihrer Erstellung enthalten, in dem Messwertspeicher des Handgeräts abgelegt wird. In dem Messwertspeicher eines erfindungsgemäßen Geräts wird auf diese Weise eine chronologisch geordnete Folge, die Zeitkorrekturdatensätze und Messwertdatensätze enthält, erzeugt. Zeitkorrekturdatensätze und Messwertdatensätze sind in dem Messwertspeicher also nach dem Zeitpunkt der Erzeugung des betreffenden Datensatzes geordnet. Dies hat mehrere Vorteile:
- Für ein erfindungsgemäßes Handgerät genügt ein einziger Speicher, so dass sich Bauteile und damit verbundene Herstellungskosten einsparen lassen.
- Die Uhr eines erfindungsgemäßen Handgeräts kann nahezu beliebig oft verstellt werden, da die Anzahl von Zeitkorrekturdatensätzen, die zwischen Messwertdatensätzen einer Serie von Messwerten gespeichert werden können, nur durch die Größe des Messwertdatenspeichers begrenzt ist.
- Durch die Position eines Zeitkorrekturdatensatzes innerhalb einer Serie von Messwertdatensätzen ist eindeutig definiert, für welche Messwertdatensätze der betreffende Zeitkorrekturdatensatz relevant ist. Datum und Uhrzeit einer erfolgten Zeitumstellung werden deshalb für eine spätere Auswertung nicht benötigt und müssen deshalb weder erfasst noch gespeichert werden, so dass die Zeitkorrekturdatensätze eines erfindungsgemäßen Geräts eine sehr einfache Struktur haben können. Die Speicherposition eines Zeitkorrekturdatensatzes in dem Messwertspeicher genügt zusammen mit Betrag und Richtung der Verstellung der Uhr, um ein fehlerfreies Auswerten einer Serie von Messwertdatensätzen zu ermöglichen.
- Für ein erfindungsgemäßes Handgerät genügt eine sehr einfache und deshalb kostengünstige Steuereinheit, da eine Auswertung von Zeit- und Zeitkorrekturinformationen in dem Handgerät nicht erforderlich ist. Die chronologisch geordnete Folge von Datensätzen kann aus dem Messwertspeicher des Handgeräts über eine Hardwareschnittstelle ohne weiteres von einem externen Gerät gelesen und zu einem beliebigen Zeitpunkt ausgewertet werden, da alle relevanten Informationen in der chronologisch geordneten Folge von Datensätzen enthalten sind.
- Eine Auswertung kann auf einen beliebigen Bruchteil der chronologisch geordneten Folge von Messwert- und Zeitkorrekturdatensätzen beschränkt werden. Die zeitliche Relation zwischen einer beliebigen Anzahl aufeinander folgender Messwertdatensätze ist nämlich durch dazwischen angeordnete Zeitkorrekturdatensätze eindeutig dokumentiert, so dass ohne zusätzliche Information eine fehlerfreie Auswertung einer beliebigen Teilfolge von Datensätzen möglich ist. Dies erleichtert eine spätere Nutzung und erneute Auswertung der durch ein erfindungsgemäßes Handgerät gewonnen Daten mit einem externen Gerät, beispielsweise dem PC eines Arztes.
- Bei einem Teilverlust von Daten wird der verbleibende Teil der Daten nicht wertlos, da eine Auswertung auf eine beliebige Anzahl von aufeinander folgenden Datensätzen beschränkt werden kann.

Eine vorteilhafte Weiterbildung der Erfindung sieht vor, dass die Messwertdatensätze und die Zeitkorrekturdatensätze dieselbe Länge haben. Auf diese Weise lassen sich die Architektur und Verwaltung des Messwertspeichers vereinfachen, da in dem Messwertspeicher stets nur Zeichenketten gleicher Länge als Datensätze gespeichert werden müssen.

Zeitkorrekturdatensätze und Messwertdatensätze können beispielsweise durch ein besonderes Datenfeld oder ein Flag von einander unterschieden werden. Bevorzugt, ist jedoch, dass die Zeitkorrekturdatensätze durch eine Markierung als Zeitkorrekturdatensätze gekennzeichnet sind, deren Beginn sich in Bezug auf den Anfang des Datensatzes in einer Position befindet, in der bei einem Messwertdatensatz ein Feld, in dem der Messwert steht, beginnt. Auf diese Weise können die Datensätze eine vorteilhaft kurze Länge haben, da kein zusätzliches Feld zu ihrer Unterscheidung benötigt wird. Die erfindungsgemäß vorgesehene Markierung kann nämlich beispielsweise in einer Zeichen- oder Bitfolge bestehen, die in dem Messwertfeld einer Messwertdatensatzes einen physiologisch unmöglichen Wert darstellen würde. Besonders vorteilhaft ist dabei, als Markierung eine Zeichenfolge zu verwenden, die mit der Ziffer 9, insbesondere mit 99, beginnt.

Indem die Markierung mit einer Bitfolge beginnt, welche die Ziffer 9 repräsentiert, kann beispielsweise die Ziffernfolge 99 oder 999 in einem Datenfeld, das bei einem Messwertdatensatz einen Messwert enthält, als Markierung für einen Zeitkorrekturdatensatz verwendet werden, da entsprechende Konzentrationswerte praktisch nicht vorkommen.

Bevorzugt ist ferner, dass die Markierungen der Zeitkorrekturdatensätze dieselbe Länge wie das den Messwert enthaltende Feld eines Messwertdatensatzes haben. Auch wenn beispielsweise bei dem vorstehend beschriebenen Beispiel zwei Ziffern 9 gefolgt von einer beliebigen dritten Ziffer als Markierung für einen Zeitkorrekturdatensatz an sich ausreichen könnten, lässt sich durch eine größere Anzahl von Zeichen beziehungsweise Ziffern eine erhöhte Sicherheit gegen Schreib- und Lesefehler erreichen.

Bevorzugt steht die Markierung, durch welche Zeitkorrekturdatensätze als solche gekennzeichnet sind, am Beginn eines Zeitkorrekturdatensatzes. Auf diese Weise können Zeitkorrekturdatensätze als solche erkannt werden.

Weitere Einzelheiten und Vorteile der Erfindung werden an einem Ausführungsbeispiel unter Bezugnahe auf die beigefügten Zeichnungen erläutert. Es zeigen:
- Figur 1:: ein Ausführungsbeispiel eines erfindungsgemäßen Handgeräts; und
- Figur 2:: die Datenstruktur der von dem Handgerät erzeugten Datensätze.

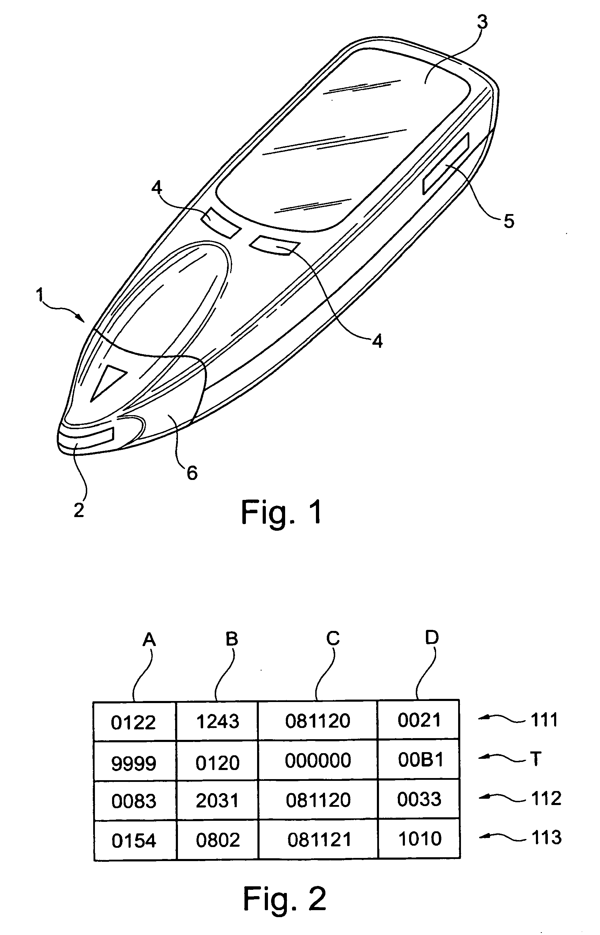

Figur 1 zeigt ein Ausführungsbeispiel eines medizinischen Handgeräts 1 zur Bestimmung einer Analytkonzentration in einer menschlichen oder tierischen Körperflüssigkeitsprobe. Bei dem dargestellten Beispiel ist das Handgerät 1 ein Glucometer, das von Diabetikern zur Bestimmung der Glukosekonzentration einer Probe vom Blut oder interstitieller Flüssigkeit verwendet werden kann.

Zur Messung wird eine Körperflüssigkeitsprobe auf einen Testfeldabschnitt eines Trägerbands 2 aufgebracht und nach einem geeignet bemessenen Bandtransportschritt die Analytkonzentration in der aufgebrachten Probe mit einer Messeinheit im Inneren des Geräts 1 gemessen, beispielsweise fotometrisch oder elektrochemisch. Möglich ist es auch, das Handgerät 1 so auszubilden, dass es beispielsweise mit Verbrauchsmaterial in Form von Teststreifen eine Konzentrationsbestimmung vornehmen kann. Testelemente können beispielsweise in einem Magazin im Inneren des Geräts gespeichert oder durch eine Gehäuseöffnung mit einer aufgebrachten Probe eingeführt werden. Um frisches Verbrauchsmaterial, beispielsweise eine Kassette mit Trägerband 2, einzulegen oder Batterien in einem Batteriefach auszutauschen, ist ein Gehäuseteil 6 abnehmbar ausgebildet.

Messergebnisse der Analytkonzentration werden mit einer Anzeigeeinrichtung 3, bevorzugt eine Flüssigkristallanzeige, beispielsweise einem Segmentdisplay, angezeigt. Gewonnene Messwerte können dabei zusammen mit Datum und Uhrzeit, die von einer internen Uhr des Handgeräts 1 geliefert werden, angezeigt werden. Eine Steuereinheit des Handgeräts 1, beispielsweise ein Mikroprozessor, erzeugt aus einem Messwert sowie Datum und Uhrzeit der dem Datum zugrunde liegenden Messung jeweils einen Messwertdatensatz und schreibt diesen in einen Messwertspeicher, der über eine Hardwareschnittstelle 5 von einem externen Gerät auslesbar ist.

Bei dem dargestellten Ausführungsbeispiel ist die Hardwareschnittstelle für eine Steckverbindung ausgelegt. Möglich ist es beispielsweise auch, die Hardwareschnittstelle für eine drahtlose Datenübertragung auszubilden.

Zur Bedienung des Geräts 1 und insbesondere zum Stellen der Uhr sind vom Benutzer betätigbare Bedienungselemente 4 vorgesehen. Bei einem Verstellen der internen Uhr, erzeugt die Steuereinheit des Geräts 1 einen Zeitkorrekturdatensatz, der Betrag und Richtung der Verstellung angibt, und schreibt diesen in den Messwertspeicher, so dass in dem Messwertspeicher eine chronologisch geordnete Folge, die Zeitkorrekturdatensätze und Messwertdatensätze enthält, erzeugt wird. Die chronologische Ordnung bezieht sich dabei jeweils auf den Zeitpunkt, zu dem der betreffende Datensatz erzeugt wurde, und ergibt sich von selbst, indem die Datensätze einfach hintereinander in dem Messwertspeicher abgelegt werden.

Figur 2 zeigt die Struktur der in dem Messwertspeicher als Folge gespeicherten Datensätze. Als Ausschnitt der Folge sind dabei schematisch vier aufeinander folgende Datensätze M1, T, M2 und M3 als Ausschnitt der in einem Messwertspeicher abgelegten Folge dargestellt. Die Datensätze M1, M2 und M3 sind Messwertdatensätze, der Datensatz T ein Zeitkorrekturdatensatz. Die Datensätze M1, M2, M3 und T haben jeweils dieselbe Länge und weisen jeweils vier Datenfelder A, B, C, D auf.

Das erste Datenfeld A hat eine Länge von vier Zeichen und enthält bei den Messwertdatensätzen M1, M2, M3 einen Messwert einer Glukosekonzentration, beispielsweise in mg/dl. Bei dem Zeitkorrekturdatensatz T enthält das Datenfeld D den Eintrag 9999 und ist dadurch als Zeitkorrekturdatensatz gekennzeichnet.

Das zweite Datenfeld B hat ebenfalls die Länge von vier Zeichen und gibt bei den Messwertdatensätzen M1, M2, M3 die Tageszeit der Messung an, wobei die ersten beiden Ziffern die Stunde von 0 bis 24 und die letzten beiden Ziffern die dazugehörende Minutenangabe von 0 bis 59 angeben. Bei dem Zeitkorrekturdatensatz T enthält das Datenfeld B den Betrag einer erfolgten Verstellung der Tageszeit.

Bei dem dargestellten Ausführungsbeispiel wird in dem Datenfeld D des Zeitkorrekturdatensatzes T die Anzahl der Minuten, um welche die Tageszeit verstellt wurde, angegeben, so dass das Datenfeld B bei einem Zeitkorrekturdatensatz einen Eintrag zwischen 0 und der maximalen Anzahl der in einem Zeitraum von 24 Stunden enthaltenen Minuten, also 1440, enthalten. Prinzipiell ist es jedoch auch möglich, bei einem Zeitkorrekturdatensatz an der entsprechenden Stelle des Datenfeldes jeweils die Anzahl der Stunden bzw. Minuten anzugeben, also mit den ersten beiden Ziffern des Datenfeldes B die Anzahl, um die der Stundenwert der Uhr verstellt wurde, und mit der letzten beiden Ziffern des Datenfeldes B die Anzahl der Minuten, um welche die Minutenanzeige der Uhr verstellt wurde.

Bei dem dargestellten Beispiel des Zeitkorrekturdatensatzes T wurde die Uhr des Handgeräts 1 um 2 Stunden verstellt, so dass in dem Datenfeld B dem entsprechend 0120 eingetragen ist, um anzugeben, dass die Uhr um 120 Minuten verstellt wurde.

Das dritte Datenfeld C der Datensätze gibt bei einem Messwertdatensatz das Datum der Messwerte an, wobei bei dem dargestellten Ausführungsbeispiel die ersten beiden Ziffern das Jahr, die folgenden beiden Ziffern den Monat und die letzten beiden Ziffern den Tag angeben. Bei einem Zeitkorrekturdatensatz können die letzten beiden Ziffern angeben, um wie viele Tage, verstellt wurde, die beiden vorletzten Ziffern angeben, um wie viele Monate verstellt wurde, und die ersten beiden Ziffern angeben, um wie viele Jahre der betreffende Teil des Datums verstellt wurde.

Das vierte Feld D der Datensätze enthält bei dem erläuterten Ausführungsbeispiel verschieden Flags, die jeweils angeben, ob die bei einem Zeitkorrekturdatensatz in den Datenfeldern B und C Einträge positiv oder negativ sind, geben also die Richtung der erfolgten Verstellung der Uhr an. Bei dem Datensatz T gibt beispielsweise das Zeichen B als Flag an, dass die Uhr zurückgestellt wurde. Bevorzugt enthält das Feld D zusätzlich ein Prüfbit oder eine Prüfziffer, um eine Kontrolle des Datensatzes auf eventuelle Schreib- oder Lesefehler zu ermöglichen. Zusätzlich kann das Feld D einen Eintrag enthalten, der angibt, ob der Messwert des betreffenden Messwertsdatensatzes einen vorgegeben Schwellenwert über- oder unterschreitet.

Das Datenfeld B wird bei einem Messwertdatensatz als Tageszeitfeld und bei einem Zeitkorrekturdatensatz als Tageszeitkorrekturfeld bezeichnet. Entsprechend wird das Datenfeld C bei einem Messwertdatensatz als Datumsfeld und bei einem Zeitkorrekturdatensatz als Datumskorrekturfeld bezeichnet.

Es kann vorkommen, dass bei einer Verstellung der Uhr nicht festgestellt werden kann, um wie viel die Uhr verstellt wurde. Dieser Fall kann beispielsweise bei der erstmaligen in Betriebnahme des Geräts oder nach einem längeren Stromausfall, beispielsweise einem Batteriewechsel, auftreten. In einem solchen Fall schreibt die Steuereinheit in den Speicher einen Sonderdatensatz, der bevorzugt dieselbe Länge und Struktur wie die Messwert- und Zeitkorrekturdatensätze hat. Ein solcher Sonderdatensatz ist durch eine besondere Markierung von einem Zeitkorrekturdatensatz unterscheidbar, die beispielsweise in dem Flagfeld D stehen kann.

### Bezugszeichen

- 1: Handgerät
- 2: Trägerband
- 3: Anzeigeeinrichtung
- 4: Bedienungselement
- 5: Hardwareschnittstelle
- M1, M2, M3: Messwertdatensatz
- T: Zeitkorrekturdatensatz
- A, B, C, D: Datenfeld

## Patentansprüche

1. Handgerät zur Messung einer Analytkonzentration in einer Körperflüssigkeitsprobe, mit
einer Anzeigeeinrichtung (3) zum Anzeigen von Messwerten,
einem Messwertspeicher zum Speichern von Messwerten,
einer Messeinheit zum Erzeugen von Messwerten durch Messung der Analytkonzentration von Körperflüssigkeitsproben,
einer Uhr, um Datum und Uhrzeit zu liefern,
einer Steuereinheit, die Messwertdatensätze (M1, M2, M3), die jeweils einen von der Messeinheit gelieferten Messwert sowie Datum und Uhrzeit der dem Messwert zugrunde liegenden Messung enthalten, erzeugt und in den Messwertspeicher schreibt,
benutzerbetätigbaren Bedienungselementen (4) zum Stellen der Uhr, wobei die Steuereinheit bei einem Verstellen der Uhr einen Zeitkorrekturdatensatz (T) erzeugt, der Betrag und Richtung der Verstellung angibt, **dadurch gekennzeichnet, dass**
die Steuereinheit Zeitkorrekturdatensätze (T) in den Messwertspeicher schreibt, so dass in dem Messwertspeicher eine chronologisch geordnete Folge, die Zeitkorrekturdatensätze (T) und Messwertdatensätze (M1, M2, M3) enthält, erzeugt wird.

2. Handgerät nach Anspruch 1, **dadurch gekennzeichnet, dass** die Messwertdatensätze (M1, M2, M3) und die Zeitkorrekturdatensätze (T) dieselbe Länge haben.

3. Handgerät nach einem der vorstehenden Ansprüche, **dadurch gekennzeichnet, dass** die Zeitkorrekturdatensätze (T) durch eine Markierung als Zeitkorrekturdatensätze (T) gekennzeichnet sind, deren Beginn sich in Bezug auf den Anfang des Datensatzes (T) in einer Position befindet, in der bei einem Messwertdatensatz (M1, M2, M3) ein Feld, in dem der Messwert angegeben ist, beginnt.

4. Handgerät nach Anspruch 3, **dadurch gekennzeichnet, dass** die Markierung eines Zeitkorrekturdatensatzes (T) dieselbe Länge wie das den Messwert enthaltende Feld eines Messwertdatensatzes (M1, M2, M3) hat.

5. Handgerät nach einem der vorstehenden Ansprüche, **dadurch gekennzeichnet, dass** die Markierung mit einer Bitfolge beginnt, welche die Ziffer 9 repräsentiert.

6. Handgerät nach einem der vorstehenden Ansprüche, **dadurch gekennzeichnet, dass** die Markierung am Beginn eines Zeitkorrekturdatensatzes (T) steht.

7. Handgerät nach einem der vorstehenden Ansprüche, **dadurch gekennzeichnet, dass** die Messwertdatensätze (M1, M2, M3) ein Zeitfeld (B), in dem die Uhrzeit der Messung angegeben ist, enthalten, wobei die Zeitkorrekturdatensätze (T) an der Stelle, an der bei Messwertdatensätze (M1, M2, M3) das Zeitfeld (B) ist, ein Tageszeitkorrekturfeld (B) enthalten, in dem angegeben ist, um wie viele Minuten die Tageszeit durch Verstellen der Uhr geändert wurde.

8. Handgerät nach einem der vorstehenden Ansprüche, **dadurch gekennzeichnet, dass** die Messwertdatensätze (M1, M2, M3) ein Datumsfeld (C) enthalten, in dem das Datum der Messung angegeben ist, wobei die Zeitkorrekturdatensätze (T) an der Stelle, an der bei Messwertdatensätzen (M1, M2, M3) das Datumsfeld (C) ist, ein Datumskorrekturfeld (C) enthalten, in dem angegeben ist, um wie viele Tage das Datum durch Verstellen der Uhr geändert wurde

9. Handgerät nach einem der vorstehenden Ansprüche, **dadurch gekennzeichnet, dass** der Zeitkorrekturdatensatz (T) ein Flagfeld (D) enthält, in dem die Richtung der Verstellung angegeben ist.

10. Handgerät nach Anspruch 9, **dadurch gekennzeichnet, dass** in dem Flagfeld (D) ein Flag die Richtung der Verstellung der Minuten und ein weiteres Flag die Richtung der Verstellung einer weiteren Zeiteinheit, vorzugsweise von Tagen, angibt.

11. Handgerät nach einem der vorstehenden Ansprüche, **dadurch gekennzeichnet, dass** die Steuereinheit in den Speicher einen Sonderdatensatz schreibt, wenn der Steuereinheit bei einem Stellen der Uhr keine Referenzzeit vorliegt, die durch das Stellen geändert wird und folglich weder Betrag noch Richtung einer Verstellung angeben werden können.

12. Handgerät nach Anspruch 11, **dadurch gekennzeichnet, dass** ein Sonderdatensatz durch eine Markierung von einem Zeitkorrekturdatensatz (T) unterscheidbar ist.

13. Handgerät nach Anspruch 12, **dadurch gekennzeichnet, dass** die Markierung, durch die ein Sonderdatensatz von einem Zeitkorrekturdatensatz (T) unterscheidbar ist, in dem Flagfeld steht.

14. Handgerät nach einem der vorstehenden Ansprüche, **dadurch gekennzeichnet, dass** es ein Glukometer ist.

15. Handgerät nach einem der vorstehenden Ansprüche, **dadurch gekennzeichnet, dass** es ein Batteriefach aufweist.

## Claims

1. Hand-held device for measuring an analyte concentration in a sample of a body liquid having
a display means (3) for displaying measured values;
a measured-value storage for storing measured values;
a measuring unit for generating measured values through measurements of the analyte concentration in samples of a body liquid;
a clock for supplying the date and hour of the day;
a control unit for generating measured value datasets (M1, M2, M3), each containing a measured value supplied by the measuring unit as well as the date and hour of the measurement by which the measured value was obtained, and for writing them into the measured-value storage;
operating elements (4) that can be actuated by the user to set the clock, in which case the control unit will generate a time-correction dataset (T) indicating the amount and direction of the setting effected, **characterized in that**
the control unit writes time-correction datasets (T) into the measured-value storage so that a chronologically ordered sequence is generated containing time-correction datasets (T) and measured value datasets (M1, M2, M3).

2. The hand-held device as defined in Claim 1, **characterized in that** the measured value datasets (M1, M2, M3) and the time-correction datasets (T) have the same length.

3. The hand-held device as defined in any of the preceding claims, **characterized in that** the time-correction datasets (T) are identified as time-correction datasets (T) by a marking that begins, related to the beginning of the dataset (T), in the same position in which a field containing the measured value begins in a measured value dataset (M1, M2, M3).

4. The hand-held device as defined in claim 3, **characterized in that** the marking of a time-correction dataset (T) has the same length as the field of a measured value dataset (M1, M2, M3) that contains the measured value.

5. The hand-held device as defined in any of the preceding claims, **characterized in that** the marking begins by a bit string that represents the character 9.

6. The hand-held device as defined in any of the preceding claims, **characterized in that** the marking is positioned at the beginning of a time-correction dataset (T).

7. The hand-held device as defined in any of the preceding claims, **characterized in that** the measured value datasets (M1, M2, M3) contain a time field (B) that indicates the hour of the day when the measurement was taken, the time-correction datasets (T) containing, at the place where the time field (B) is found in the measured value datasets (M1, M2, M3), a time-correction field (B) that indicates the number of minutes by which the hour of the day was changed by a clock setting operation.

8. The hand-held device as defined in any of the preceding claims, **characterized in that** the measured value datasets (M1, M2, M3) contain a date field (C) that indicates the date on which the measurement was taken, the time-correction datasets (T) containing, at the place where the date field (C) is found in the measured value datasets (M1, M2, M3), a date-correction field (C) that indicates the number of days by which the date was changed by a clock setting operation.

9. The hand-held device as defined in any of the preceding claims, **characterized in that** the time-correction dataset (T) contains a flag field (D) that indicates the direction in which the setting was made.

10. The hand-held device as defined in Claim 9, **characterized in that** a flag in the flag field (D) indicates the direction of the setting in minutes, while a further flag indicates the direction of the setting in another time unit, preferably in days.

11. The hand-held device as defined in any of the preceding claims, **characterized in that** the control unit writes a special dataset into the storage when at the time the clock is set no reference time is available that would be altered by the setting operation and when, consequently, neither the amount nor the direction of a setting can be indicated.

12. The hand-held device as defined in Claim 11, **characterized in that** a special dataset can be distinguished from a time-correction dataset (T) by a marking.

13. The hand-held device as defined in Claim 12, **characterized in that** the marking by which a special dataset can be distinguished from a time-correction dataset (T) is positioned in the flag field.

14. The hand-held device as defined in any of the preceding claims, **characterized in that** the device is a glucometer.

15. The hand-held device as defined in any of the preceding claims, **characterized in that** the device comprises a battery compartment.

## Revendications

1. Appareil manuel destiné à la mesure d'une concentration en analytes dans un échantillon de liquide corporel, comprenant
un dispositif d'affichage (3) destiné à afficher des valeurs de mesure,
une mémoire de valeurs de mesure destinée à mémoriser des valeurs de mesure,
une unité de mesure destinée à générer des valeurs de mesure par mesure de la concentration en analytes d'échantillons de liquide corporel, une horloge afin de fournir la date et l'heure,
une unité de commande qui génère des jeux de données de valeurs de mesure (M1, M2, M3), lesquels contiennent respectivement une valeur de mesure fournie par l'unité de mesure ainsi que la date et l'heure de la mesure à la base de la valeur de mesure, et les inscrit dans la mémoire de valeurs de mesure,
des éléments de commande (4) pouvant être commandés par un utilisateur, destinés à régler l'horloge, l'unité de commande, lors d'un réglage de l'horloge, générant un jeu de données de correction de temps (T) qui indique le montant et le sens du réglage, **caractérisé en ce que**
l'unité de commande inscrit des jeux de données de correction de temps (T) dans la mémoire de valeurs de mesure de sorte qu'une séquence classée chronologiquement, laquelle contient les jeux de données de correction de temps (T) et les jeux de données de valeurs de mesure (M1, M2, M3), est générée dans la mémoire de valeurs de mesure.

2. Appareil manuel selon la revendication 1, **caractérisé en ce que** les jeux de données de valeurs de mesure (M1, M2, M3) et les jeux de données de correction de temps (T) ont la même longueur.

3. Appareil manuel selon l'une quelconque des revendications précédentes, **caractérisé en ce que** les jeux de données de correction de temps (T) sont **caractérisés par** un marquage en tant que jeux de données de correction de temps (T), dont le début, par rapport au début du jeu de données (T), se trouve dans une position dans laquelle, dans un jeu de données de valeurs de mesure (M1, M2, M3), un champ commence, dans lequel la valeur de mesure est indiquée.

4. Appareil manuel selon la revendication 3, **caractérisé en ce que** le marquage d'un jeu de données de correction de temps (T) a la même longueur que le champ, contenant la valeur de mesure, du jeu de données de valeurs de mesure (M1, M2, M3).

5. Appareil manuel selon l'une quelconque des revendications précédentes, **caractérisé en ce que** le marquage commence par une séquence de bits, laquelle représente le chiffre 9.

6. Appareil manuel selon l'une quelconque des revendications précédentes, **caractérisé en ce que** le marquage est situé au début d'un jeu de données de correction de temps (T).

7. Appareil manuel selon l'une quelconque des revendications précédentes, **caractérisé en ce que** les jeux de données de valeurs de mesure (M1, M2, M3) contiennent un champ de temps (B) dans lequel l'heure de la mesure est indiquée, les jeux de données de correction de temps (T) contenant à l'endroit, auquel se trouve le champ de temps (B) dans les jeux de données de mesure de temps (M1, M2, M3), un champ de correction de l'heure du jour (B), dans lequel il est indiqué de combien de minutes l'heure du jour a été modifiée en raison du réglage de l'horloge.

8. Appareil manuel selon l'une quelconque des revendications précédentes, **caractérisé en ce que** les jeux de données de valeurs de mesure (M1, M2, M3) contiennent un champ de date (C), dans lequel la date de la mesure est indiquée, les jeux de données de correction de temps (T) contenant à l'endroit, auquel se trouve le champ de date (C) dans les jeux de données de mesure de temps (M1, M2, M3), un champ de correction de date (C), dans lequel il est indiqué de combien de jours la date a été modifiée en raison du réglage de l'horloge.

9. Appareil manuel selon l'une quelconque des revendications précédentes, **caractérisé en ce que** le jeu de données de correction de temps (T) contient un champ indicateur (D), dans lequel le sens du réglage est indiqué.

10. Appareil manuel selon la revendication 9, **caractérisé en ce que** dans le champ indicateur (D) un indicateur indique le sens du réglage des minutes et un indicateur supplémentaire indique le sens de réglage d'une unité de temps supplémentaire, de préférence de jours.

11. Appareil manuel selon l'une quelconque des revendications précédentes, **caractérisé en ce que** l'unité de commande inscrit dans la mémoire un jeu de données spécial lorsque, lors d'un réglage de l'horloge, aucun temps de référence n'est présent pour l'unité de commande, lequel temps de référence est changé en raison du réglage et qu'en conséquence ni le montant ni le sens d'un réglage ne peuvent être indiqués.

12. Appareil manuel selon la revendication 11, **caractérisé en ce qu'**un jeu de données spécial peut être distingué d'un jeu de données de correction de temps (T) par un marquage.

13. Appareil manuel selon la revendication 12, **caractérisé en ce que** le marquage, au moyen duquel un jeu de données spécial peut être distingué d'un jeu de données de correction de temps (T), se trouve dans le champ indicateur.

14. Appareil manuel selon l'une quelconque des revendications précédentes, **caractérisé en ce que** c'est un glucomètre.

15. Appareil manuel selon l'une quelconque des revendications précédentes, **caractérisé en ce qu'**il présente un compartiment pour batterie.
